# EUROPEAN PATENT SPECIFICATION

(11) **EP 3 639 836 B1**
(45) Date of publication and mention of the grant of the patent: **15.09.2021**
(21) Application number: 18818042.6
(22) Date of filing: 13.06.2018
(51) Int. Cl.: A61K 36/71, A61K 31/192, A23L 33/105, A61P 1/16, A61P 19/02, A61P 37/02, A61P 35/00, A61P 29/00

(54) **COMPOSITION COMPRISING ISOFERULIC ACID FOR USE IN THE TREATMENT OF AUTOIMMUNE HEPATITIS**
ZUSAMMENSETZUNG ENTHALTEND ISOFERULASÄURE ZUR VERWENDUNG IN DER BEHANDLUNG VON AUTOIMMUNEHEPATITIS
COMPOSITION COMPRENANT DE L'ACIDE FÉRULIQUE DESTINÉS À ÊTRE UTILISÉS DANS LE TRAITEMENT DE L'HÉPATITE AUTO-IMMUNE

(30) Priority: 13.06.2017 CN 201710443176
(43) Date of publication of application: 22.04.2020
(73) Proprietor: Beijing Ditan Hospital, Capital Medical University, Beijing 100015 (CN)
(72) Inventor: ZENG, Hui, Beijing 100015 (CN); WANG, Xianbo, Beijing 100015 (CN); ZHU, Liuluan, Beijing 100015 (CN); LI, Rui, Beijing 100015 (CN)
(74) Representative: RGTH
(86) International application number: PCT/CN2018/091056
(87) International publication number: WO 2018/228430

(56) References cited:
- CN-A- 101 721 400
- CN-A- 101 723 944
- CN-A- 101 732 299
- CN-A- 103 974 630
- CN-A- 105 055 738
- CN-A- 106 333 944
- CN-A- 106 620 007
- KR-A- 20120 057 512
- EUN JU LEE ET AL: "HEPATOPROTECTIVE PHENYLPROPANOIDS FROM SCROPHULARIA BUERGERIANA ROOTS AGAINST CCL4-INDUCED TOXICITY: ACTION MECHANISM AND STRUCTURE-ACTIVITY RELATIONSHIP", PLANTA MEDICA, THIEME VERLAG, DE, vol. 68, no. 5, 1 January 2002 (2002-01-01), pages 407-411, XP008016716, ISSN: 0032-0943, DOI: 10.1055/S-2002-32081
- LIU I-M ET AL: "Antihyperglycemic action of isoferulic acid in streptozotocin-induced diabetic rats", BRITISH JOURNAL OF PHARMACOLOGY, WILEY-BLACKWELL, UK, vol. 129, no. 4, 1 January 2000 (2000-01-01), pages 631-636, XP003020949, ISSN: 0007-1188, DOI: 10.1038/SJ.BJP.0703082
- LIU I-MIN ET AL: "Isoferulic acid as active principle from the rhizoma of Cimicifuga dahurica to lower plasma glucose in diabetic rats", PLANTA MEDICA, THIEME VERLAG, DE, vol. 65, no. 8, 1 December 1999 (1999-12-01), pages 712-714, XP002680903, ISSN: 0032-0943, DOI: 10.1055/S-1999-14048
- A. PANOSSIAN ET AL: "Methods of phytochemical standardisation of rhizoma cimicifugae racemosae", PHYTOCHEMICAL ANALYSIS, vol. 17, no. 3, 1 May 2006 (2006-05-01), pages 208-208, XP055679978, ISSN: 0958-0344, DOI: 10.1002/pca.901
- Liu, Ying et al.: "Study on Cimicifuga Quality Standard", Zhongcaoyao - Chinese Herbal Medicines, vol. 36, no. 9, 30 September 2005 (2005-09-30), pages 1402 (126)-1404 (128), XP009518174, China
- HUANG Gui-ping: "Screening of potent active components of Cimicifugae Rhizoma for treating Hepatitis B virus", Chinese Journal of Experimental Traditional Medicine Formulae, vol. 19, no. 21, 30 November 2013 (2013-11-30), pages 231-235, XP009518180, ISSN: 1005-9903

## Description

### TECHNICAL FIELD

The present invention relates to the field of medicine, in particular to the use of isoferulic acid, Chinese medicine extracts containing isoferulic acid, Cimicifugae rhizoma and the use in preparing a medicine or a functional health product for autoimmune diseases.

### BACKGROUND OF THE INVENTION

The activation of innate immune cells and T lymphocytes is the key pathogenesis of autoimmune diseases. Typical autoimmune diseases are represented by autoimmune hepatitis and rheumatoid arthritis. These diseases are usually developed through stimulating the body by a variety of pathogenic factors, thus, causing the body's immune disorders. The development of new drugs or health products from the perspective of regulating the body's immunity will be a breakthrough for treating this type of diseases. Therefore, the development of immunomodulatory drugs or functional health products from the traditional medicine resources of the motherland has broad prospects.

Isoferulic acid is the main active monomer ingredient in the extracts of Chinese medicinal materials such as Cimicifugae rhizoma. The chemical name of isoferulic acid is 3-hydroxy-4-methoxycinnamic acid, also known as citrate or caffeic acid-4-methyl ether. The chemical formula is C₁₀H₁₀O₄, which has antioxidation and blood sugar lowering effects.

KR 2012 0057512 A discloses pharmaceutical compositions for preventing or treating liver disorders, especially alcohol liver diseases (ALDs) comprising Oenanthe javanica extracts as an active ingredient. Its content discloses, also to a water parsley extract that has alcohol decomposition abilities.

### SUMMARY OF THE INVENTION

One of the objects of the present invention is to provide isoferulic acid, Chinese medicine extracts containing isoferulic acid, Cimicifugae rhizoma, and the use thereof. The present invention provides the use of isoferulic acid, Chinese medicine extracts containing isoferulic acid, and Cimicifugae rhizoma in the preparation of a medicament or a functional health product for autoimmune diseases.

In the above applications, the autoimmune disease is autoimmune hepatitis.

The present invention also provides the use of isoferulic acid, Chinese medicine extracts containing isoferulic acid, and Cimicifugae rhizoma in the preparation of a medicament or a functional health product for inhibiting inflammatory cytokines, including one or more of the following inflammatory cytokines: TNF -α, IFN-γ, IL-6, IL-9, IL-12, IL-17A, IL-18, IP-10, MCP-1, MCP-3, MIP-1α, MIP-1β, MIP-2, Eotaxin, or G-CSF.

In the above application, the inflammatory cytokines may be involved in a variety of diseases caused by bacterial and/or viral infections, such as hepatitis, pneumonia, sepsis, influenza, measles, herpes simplex, etc.; they may also be involved in a variety of autoimmune diseases, such as rheumatoid arthritis, systemic vasculitis, scleroderma, multiple encephalomyelitis, etc. Isoferulic acid, Chinese medicine extracts containing isoferulic acid, and Cimicifugae rhizoma can be used in medicine or functional health products to prevent, alleviate and treat the above diseases.

Wherein, the Chinese medicine extracts containing isoferulic acid may be Cimicifugae rhizoma decoction, which is prepared by boiling the rhizomes and fibrils of Cimicifugae rhizoma to obtain the decoction.

Wherein, the isoferulic acid-containing Chinese medicine extracts may also be a Cimicifugae rhizoma total phenolic acid extract, which mainly contains isoferulic acid, ferulic acid, caffeic acid, and the like.

In the above-mentioned application, the medicine may further include a pharmaceutically acceptable carrier in addition to isoferulic acid, a Chinese medicine extract containing isoferulic acid, or Cimicifugae rhizoma. Pharmaceutically acceptable carriers include, but are not limited to, excipients, binders, lubricants, fillers, disintegrants, emulsifiers, stabilizers, colorants, flavoring agents, preservatives, etc. The medicine can be prepared into any common dosage form known in the present pharmaceutical field, including, but not limited to, oral dosage forms such as tablets, capsules, granules, and pills, and non-oral dosage forms such as injections and lyophilizates.

In the above application, in addition to having isoferulic acid, Chinese medicine extract containing isoferulic acid, or Cimicifugae rhizoma as an active ingredient, the functional health product can also have common ingredients, including but not limited to nutrients, vitamins, minerals, aromatizer, colorant, tackifier, pH adjuster, stabilizer, preservative, etc. The functional health products can be consumed alone or used in combination with existing medicines or health products.

Another object of the present invention is to provide a pharmaceutical composition containing isoferulic acid or an isoferulic acid-containing Chinese medicine extract, or a Chinese medicine composition containing Cimicifugae rhizoma in the preparation of a medicament for an autoimmune disease.

In the above application, the autoimmune disease is autoimmune hepatitis.

Wherein, the Chinese medicine extracts containing isoferulic acid may be Cimicifugae rhizoma decoction, which is prepared by boiling the rhizomes and fibrils of Cimicifugae rhizoma to obtain the decoction.

Wherein, the isoferulic acid-containing Chinese medicine extracts may also be a Cimicifugae rhizoma total phenolic acid extract, which mainly contains isoferulic acid, ferulic acid, caffeic acid, and the like.

In the above-mentioned application, the pharmaceutical composition may further include a pharmaceutically acceptable carrier in addition to isoferulic acid, a Chinese medicine extract containing isoferulic acid, or Cimicifugae rhizoma. Pharmaceutically acceptable carriers include, but are not limited to, excipients, binders, lubricants, fillers, disintegrants, emulsifiers, stabilizers, colorants, flavoring agents, preservatives, etc. The pharmaceutical composition may be administered alone or in combination with existing drugs for autoimmune diseases, and can be prepared into any common dosage form known in the present pharmaceutical field, including, but not limited to, oral dosage forms such as tablets, capsules, granules, and pills, and non-oral dosage forms such as injections and lyophilizates.

In the above application, the Chinese medicine composition may be a compatible medicine formed by Cimicifugae rhizoma and other traditional Chinese medicine ingredients, such as Angelica, Bupleurum, Chenpi and the like. The Chinese medicine composition can be administered alone or in combination with existing drugs for autoimmune diseases. It can be prepared into any common dosage form known in the existing traditional Chinese medicine field, including but not limited to decoctions, liquors, tea, lotion, pill, powder, ointment, elixir, tablet, lozenge and so on.

The third object of the present invention is to provide a pharmaceutical composition.

The pharmaceutical composition provided by the present invention may be a pharmaceutical composition for an autoimmune disease, which contains isoferulic acid, an isoferulic acid-containing Chinese medicine extract or Cimicifugae rhizoma as an active ingredient.

In the above pharmaceutical composition for autoimmune diseases, the autoimmune disease is autoimmune hepatitis.

The present invention also provides a pharmaceutical composition having isoferulic acid, Chinese medicine extracts containing isoferulic acid, or Cimicifugae rhizoma as an active ingredient for the inhibition of inflammatory cytokines, including one or more of the following inflammatory cytokines: TNF -α, IFN-γ, IL-6, IL-9, IL-12, IL-17A, IL-18, IP-10, MCP-1, MCP-3, MIP-1α, MIP-1β, MIP-2, Eotaxin, or G-CSF.

Wherein, the Chinese medicine extracts containing isoferulic acid may be Cimicifugae rhizoma decoction, which is prepared by boiling the rhizomes and fibrils of Cimicifugae rhizoma to obtain the decoction.

Wherein, the isoferulic acid-containing Chinese medicine extracts may also be a Cimicifugae rhizoma total phenolic acid extract, which mainly contains isoferulic acid, ferulic acid, caffeic acid, and the like.

The above pharmaceutical composition may further include a pharmaceutically acceptable carrier in addition to active ingredient. Pharmaceutically acceptable carriers include, but are not limited to, excipients, binders, lubricants, fillers, disintegrants, emulsifiers, stabilizers, colorants, flavoring agents, preservatives, etc. The pharmaceutical composition may be administered alone or in combination with existing drugs for autoimmune diseases, and can be prepared into any common dosage form known in the present pharmaceutical field, including, but not limited to, oral dosage forms such as tablets, capsules, granules, and pills, and non-oral dosage forms such as injections and lyophilizates.

In the present invention, through the animal model experiment of Concanavalin A (ConA), it is found that: isoferulic acid, Chinese medicine extracts and pharmaceutical compositions containing isoferulic acid are protective against acute immune liver damage caused by concanavalin A. It can significantly improve the survival rate, liver function level, liver pathological damage and inflammatory cytokine secretion level of the animal models of autoimmune hepatitis, indicating that it can be used to prepare medicines or functional health products for treating or alleviating autoimmune hepatitis.

In the present invention, through Collagen induced arthritis (CIA) animal model experiments, it is found that isoferulic acid and Chinese medicine (such as Cimicifugae rhizoma) containing isoferulic acid have protective effects on multiple joint damages caused by CIA. It can significantly reduce the incidence of CIA animal models and the average index of arthritis, and improve the destruction of bone structure, reduce the secretion of anti-type II collagen antibodies and a variety of inflammatory cytokines, indicating that it has an exact anti-arthritis effect, and can be used to for the preparation of medicines or functional health products for treating or relieving rheumatoid arthritis symptoms.

The present invention also shows that isoferulic acid and Chinese medicine extracts and pharmaceutical compositions containing isoferulic acid can significantly inhibit a variety of inflammatory cytokines at the same time, including one or more of the following inflammatory cytokines: TNF -α, IFN-γ, IL-6, IL-9, IL-12, IL-17A, IL-18, IP-10, MCP-1, MCP-3, MIP-1α, MIP-1β, MIP-2, Eotaxin, or G-CSF.

In summary, the present invention provides new uses of isoferulic acid, Chinese medicine extracts, pharmaceutical compositions, and Chinese medicine compositions containing isoferulic acid. Because of the extensive immunosuppressive effect, they have been developed for as drugs or functional health products for autoimmune diseases. Not only can isoferulic acid be produced through chemical synthesis or extraction, Cimicifugae rhizoma extract or compatible Chinese medicine containing Cimicifugae rhizoma can also be directly used. The preparation is simple and there is extensive source of raw materials. The cost is low and it has a broad market application value.

### BRIEF DESCRIPTION OF THE DRAWINGS

- FIG. 1: shows a chart of mice survival rate in each group in Example 1.
- FIG. 2: shows a micrograph (HE staining) of liver tissue of the mouse model of each group in Example 2.
- FIG. 3: shows a picture of a paw joint of the mouse in each group in Example 3.
- FIG. 4: shows a Micro-CT image of a right hind limb knee joint, ankle joint, and toe joint of the mouse in each group in Example 6.

### EMBODIMENTS

The present invention is described in detail through preparation examples and experimental examples to make the features and advantages of the present invention clearer. However, it should be noted that the preparation examples and experimental examples are used to understand the concept of the present invention, and the scope of the present invention is not limited to the preparation examples and experimental examples listed herein.

Unless otherwise specified, the experimental methods used in the following Preparation Examples and Experimental Examples are conventional methods. The materials and reagents used can be obtained from commercial sources unless otherwise specified.

### Preparation Example 1. Preparation of medicament

Cimicifugae rhizoma was purchased from Tongrentang Qianmen Head Office. 12g of Cimicifugae rhizoma was immersed in distilled water for 1 ~ 2 h. Decocting for 10 minutes with high heat and then gently for 1 h. Concentrate the decoction to 75 mL. The concentration of Cimicifugae rhizoma decoction was about 0.16 g/mL. The decoction was divided into 5 mL centrifuge tubes and stored at -20°C until use. Cimicifugae rhizoma, Angelicae sinensis radix, Bupleuri radix and Citri Reticulatae pericarpium were purchased from Tongrentang Qianmen Head Office. 12g of Cimicifugae rhizoma with 6g of Angelicae sinensis radix, 12g of Bupleuri radix and 12g of Citri Reticulatae pericarpium were immersed in distilled water for 1 ~ 2 h. Decocting for 10 minutes with high heat and then gently for 1 h. The decoction containing the extract of Cimicifugae rhizoma, Angelicae sinensis radix, Bupleuri radix and Citri Reticulatae pericarpium was concentrated to 75 mL, with a concentration of about 0.56 g / mL, then divided into 5 mL centrifuge tubes and stored at -20 ° C to be used for the Cimicifugae rhizoma compatibility group.

### Preparation Example 2. Extraction of Cimicifugae rhizoma Total Phenolic Acid

Cimicifugae rhizoma total phenolic acid extraction was based on the reference (Huang Guiping, Li Cunyu, Li Hongyang, Zhi Xinglei, Li Hemin, Liu Lanping, Peng Guoping. Optimization of Extraction Process of Phenolic Acids in Cimicifugae rhizoma. Journal of Liaoning University of Traditional Chinese Medicine, 2014, 4: 50-52). Took 75 mL of Cimicifugae rhizoma decoction and added 95% ethanol to 1000 mL, precipitated overnight, and separated the supernatant. The supernatant was distilled under reduced pressure to sufficiently remove ethanol and excess water and concentrated to 75 mL to obtain a Cimicifugae rhizoma extract. At room temperature, organic solvent ethyl acetate was used to extract 5 times, stirred, extracted, and centrifuged to obtain the supernatant, and then extracted with 5% sodium carbonate. The solution was adjusted to pH 3 with diluted hydrochloric acid and extracted with an equal amount of ethyl acetate. After extraction of 5 times, the solution was recovered under reduced pressure, and Cimicifugae rhizoma total phenolic acid extract was obtained, and the volume was adjusted to 75 mL. Isoferulic acid, ferulic acid, and caffeic acid standards (all purchased from Tianjin Yifang Technology Co., Ltd.) were used as controls. The content of each component was identified by HPLC method as shown in Table 1.

**Table 1. Contents of three major phenolic acids**

| | Isoferulic acid | Ferulic acid | Caffeic acid |
|---|---|---|---|
| Phenolic acid extract | 1.406 mg/g | 1.306 mg/g | 0.369 mg/g |

### Experimental Example 1. Effect on survival rate of hepatitis mouse model of ConA

Test materials: ConA was purchased from Sigma; isoferulic acid was purchased from Tianjin Fangfang Technology Co., Ltd.

Animals: C57BL/6 mice, purchased from Beijing Huafukang Biotechnology Co., Ltd. Grouping: 50 C57BL/6 mice, male, 18-20 g, after 1 day of adaptive breeding, the mice were randomly divided into 5 groups of 10 in each group: ConA model group, Cimicifugae rhizoma compatibility group, Cimicifugae rhizoma group, Cimicifugae rhizoma total phenolic acid group and isoferulic acid group.

Model preparation and drug administration: 150 mg of ConA powder were weighed and dissolved in 40 mL of sterile PBS and left at room temperature for 1 to 2 hours; the solution was intermittently shaken gently to mix well. Low temperature ultrasound promoted dissolution (no more than 5 min each time) and avoided excessive foaming. After the solution was fully dissolved, the volume was adjusted to 50mL, prepared to a concentration of 3 mg/mL, and filtered under pressure with a 0.45µm filter. Mice were given a single intravenous injection at 25 mg/kg, with 100µL per mouse. 250µL of distilled water for gavage for mice in ConA model group; 250µL of Cimicifugae rhizoma water decoction obtained in Preparation Example 1 for gavage for mice in Cimicifugae rhizoma compatibility group, the amount of crude drug was 7g/kg; 250µL of Cimicifugae rhizoma water decoction obtained in Preparation Example 1 for gavage for mice in Cimicifugae rhizoma group, the amount of crude drug was 2g/kg; 250µL of Cimicifugae rhizoma total phenolic acid extract obtained in Preparation Example 2 for gavage for mice in Cimicifugae rhizoma total phenolic acid group, the amount of crude drug was 300 mg/kg; 250µL of an aqueous solution of isoferulic acid for gavage for mice in isoferulic acid group, the dose was 5 mg/kg. The mortality of the animals was observed at 96 h.

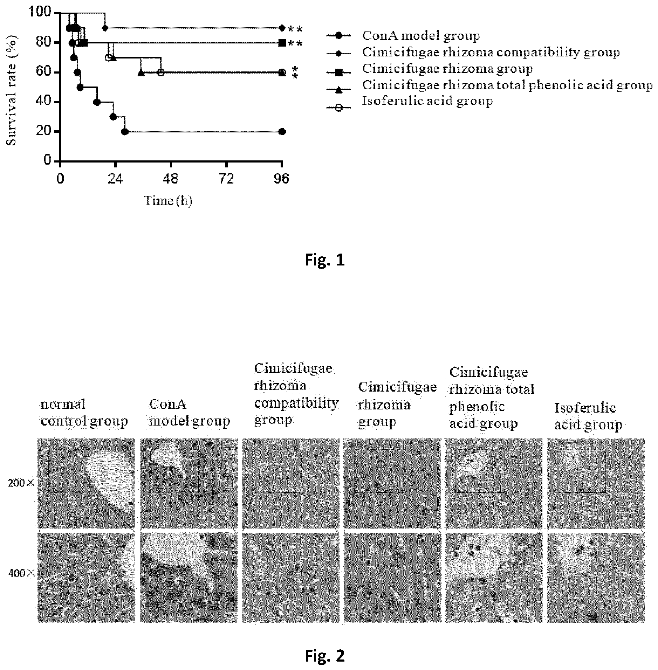

Results: as shown in Figure 1, the mice began to die 4-6 hours after ConA injection, and the deaths were concentrated within 24 hours, after which the mortality rate decreased and stabilized at 48-96 hours. The survival rate of mice in the ConA model group was 20%, and the survival rate of mice increased after drug treatment. The survival rate of mice in Cimicifugae rhizoma compatibility group was 90% (compared with the ConA model group, **, p <0.01). The survival rate of mice was 80% in Cimicifugae rhizoma group (compared to the ConA model group, **, p <0.01), and the survival rate for mice in Cimicifugae rhizoma total phenolic acid group was 60% (compared to the ConA model group, *, p <0.05). The survival rate of mice in the isoferulic acid group was 60% (compared with the ConA model group, *, p <0.05). Survival rate was statistically analyzed by Kaplan-Meier, and each of the drug treatment groups had significant differences compared with the model group. The results show that Cimicifugae rhizoma compatible medicine, Cimicifugae rhizoma extract, Cimicifugae rhizoma total phenolic acid and isoferulic acid can all improve the survival rate of ConA hepatitis mice.

### Experimental Example 2. The therapeutic effect of Cimicifugae rhizoma and its extract on liver inflammation

The test materials and animals were the same as those in Experimental Example 1. Grouping: C57BL/6 mice, male, 18-20 g, after 1 day of adaptive breeding, the mice were randomly divided into 6 groups: normal control group; ConA model group, Cimicifugae rhizoma compatibility group, Cimicifugae rhizoma group, Cimicifugae rhizoma total phenolic acid group and isoferulic acid group.

Model preparation and drug administration: except the normal control group, mice were injected intravenously once at a dose of ConA 15 mg/kg. The dosages of each drug group were the same as those of Experimental Example 1.
(1) The effects of each drug group on serum ALT and AST of ConA mice were examined. Eight mice in each group were sacrificed under anesthesia 10 hours after ConA injection. Peripheral blood was taken and serum was separated to detect ALT and AST.
(2) The effects of each drug group on liver pathological damage in ConA mice were examined. Three mice in each group were sacrificed under anesthesia 10 hours after ConA injection. The left lobe of the liver was taken, cut into a size of about 5 mm³, and placed in 10% formalin solution, and dehydrated, transparently impregnated with wax, embedded, sliced, and spread, and observed after staining;
(3) The effects of drugs in each treatment group on serum cytokine secretion in ConA mice were examined. Six mice from each group. Peripheral blood was collected 3 hours after ConA injection and serum was separated to detect the secretion of cytokines TNF-α, IL-12, IL-6 and MCP-1. Serum was separated 10 hours after ConA injection to detect cells factor IFN-γ secretion.

### Results (1): Effects of drugs on serum ALT and AST in mice with ConA hepatitis

The results are shown in Table 2. Compared with the normal group, the ALT and AST of the ConA liver injury model group were significantly different (***, p <0.001); the ALT and AST of each drug group were significantly decreased, and compared to those of the ConA model group, the differences were statistically significant (#, p <0.05; ##, p <0.01; ###, p <0.001). It indicates that the combination of Cimicifugae rhizoma compatible medicine, Cimicifugae rhizoma extract, Cimicifugae rhizoma total phenolic acid and isoferulic acid can alleviate liver injury in ConA mice.

**Table 2. Effects of Cimicifugae rhizoma compatibility medicine, Cimicifugae rhizoma extract, Cimicifugae rhizoma total phenolic acid and isoferulic acid on serum ALT and AST in a mouse model of ConA hepatitis (MEAN ± SEM)**

| | ALT(U/L) | AST(U/L) |
|---|---|---|
| normal control group | 27.5±1.5 | 89.3±9.2 |
| ConA model group | 9455±2063*** | 10726±1782*** |
| Cimicifugae rhizoma compatibility medicine group | 47.9±14.4^{###} | 189.6±23.4^{###} |
| Cimicifugae rhizoma group | 156.6±66.6^{###} | 617.2±335.9^{###} |
| Cimicifugae rhizoma total phenolic acid group | 1428±643.2^{##} | 1754±676.2^{###} |
| isoferulic acid group | 3573±1314^{#} | 4351±1588^{#} |

| | | |
|---|---|---|
| Note: * represents a statistical difference between the ConA group and the normal control group, ***, p <0.001. # represents a statistically significant difference between the medication group and the ConA group, #, p <0.05; ##, p <0.01; ###, p <0.001. | | |

### Results (2): Effects of drugs on liver pathological damage in a mouse model of ConA hepatitis

The results are shown in Figure 2. In the ConA group, the liver cells of the mice were extensively degenerated, the structure of the hepatic lobules was disordered, the central veins of the hepatocytes were evidently dilated, the central veins and hepatic sinusoids were evidently congested, and even the presence of large hepatocyte sheet necrosis and massive immune cells infiltration; compared with the ConA group, mice with Cimicifugae rhizoma compatible drugs, Cimicifugae rhizoma extract, Cimicifugae rhizoma total phenolic acid, and isoferulic acid have reduced hepatocyte degeneration and necrosis, hepatocyte sheet necrosis significantly reduced, and immune cells infiltration was reduced.

### Result (3): Effect on serum cytokines in a mouse model of ConA hepatitis

The results are shown in Table 3. Compared with the normal group, TNF-α, IL-12, MCP-1, IL-6, and T lymphocytes secreted by the natural immune cells were significantly higher in the ConA group than in the normal group; the differences (***, p <0.001) were statistically significant. Each treatment group can significantly reduce the secretion levels of cytokines IL-12, TNF-α, MCP-1, IL-6 and IFN-γ in serum (#, p <0.05; ##, p <0.01; ###, P <0.001).

**Table 3. Effects on serum cytokine secretion in a mouse model of ConA hepatitis (MEAN ± SEM)**

| | IL-12 (pg/mL) | TNF-α (pg/mL) | MCP-1 (pg/mL) | IL-6 (pg/mL) | IFN-γ (pg/mL) |
|---|---|---|---|---|---|
| Normal control group | 7.3±1.1 | 6.9±0.7 | 21±3.6 | 6.5±2.1 | 3.3±0.5 |
| ConA group | 598.7±89.4* ** | 562.3±13.22* ** | 9503±684*** | 4417±630.7* ** | 1751±234.9* ** |
| Cimicifugae rhizoma compatibilit y medicine group | 157.8±41.6^{##} | 190.4±25.8^{###} | 1712±84.9^{###} | 761.2±189.9^{##} | 300.4±71.9^{## #} |
| Cimicifugae rhizoma group | 188.8±80.9^{##} | 183.22±3.7^{###} | 3332±947.2^{# ##} | 1316±475.3^{#} | 338±151.6^{###} |
| Cimicifugae rhizoma total phenolic acid group | 238.5±20.5^{##} | 229.4±257^{###} | 5730±751.8^{##} | 2197±571.7^{#} | 440.9±93.6^{## #} |
| isoferulic acid group | 183.7±33.4^{##} | 260.4±119.5^{# ##} | 5708±145.5^{# ##} | 1422±214.2^{##} | 574±70.4^{###} |

| | | | | | |
|---|---|---|---|---|---|
| Note: * represents a statistical difference between the ConA group and the normal control group, ***, p <0.001. # represents a statistically significant difference between the medication group and the ConA group, #, p <0.05; ##, p <0.01; ###, p <0.001. | | | | | |

### Experimental Example 3. Effects of isoferulic acid and Cimicifugae rhizoma on arthritis index of CIA arthritis mice

Test materials: bovine type II collagen acetic acid solution and complete Freund's adjuvant were purchased from Chondrex company; isoferulic acid was purchased from Tianjin Yifang Technology Co., Ltd.

Animals: C57BL/6 mice, purchased from Beijing Huafukang Biotechnology Co., Ltd.

Grouping: 24 C57BL/6 mice, male, 18-20 g, after 1 day of adaptive breeding, the mice were randomly divided into 4 groups: normal control group and CIA model group, 5 mice in each group; Cimicifugae rhizoma group and isoferulic acid group, 7 mice in each group.

Model preparation and drug administration: Prepared a 2 mg/ml bovine type II collagen acetic acid solution, took 2 ml of the overnighted bovine type II collagen acetic acid solution, mixed thoroughly with 2 ml of complete Freund's adjuvant and emulsified (operation on ice), and prepared the bovine type II collagen emulsion having a final concentration of 1 mg/ml. Each mouse was injected intradermally with 0.1 ml of the emulsion. 21 days after the first immunization, mice were given a second immunization for 21 days, starting from day 0 of the second immunization, the CIA model group was given 250 µL of distilled water by gavage and the Cimicifugae rhizoma group was given 250 µL of the Cimicifugae rhizoma decoction obtained in Preparation Example 1. The crude drug dose was 2 g/kg; 250 µL of an aqueous solution of isoferulic acid was administered by gavage in the isoferulic acid group at a dose of 5 mg/kg, administered once every 3 days, and bred continuously for 21 days. Animals were scored using the joint scoring method (grades 0-4), and the assessment was based on the degree of joint redness and swelling and joint swelling and deformation. 0 point: no redness and swelling; 1 point: redness but no swelling of the joint; 2 points: mild redness and swelling of the joint; 3 points: moderate redness and swelling of the joint; 4 points: severe redness and swelling of the joint accompanied by dysfunction of the joint. Each limb wass scored separately, and the sum of the scores of each limb is the arthritis index of the mouse. The highest score is 16 points. The higher the score, the more severe the joint symptoms.

Results: As shown in Table 4, at 3 days after the second immunization, the CIA mice developed redness and mild swelling, with an average arthritis index of 0.57. As the course of the disease prolonged, the arthritis index gradually increased. At 15 days, the ankle joints and toe joints were highly swollen, the joint surface appeared congested, and the hind limbs could not bear weight. The average index of arthritis was highest and reached the plateau stage, which was 8.43. The average index of arthritis in the isoferulic acid group and the Cimicifugae rhizoma group were 4.43 and 5.71, respectively; the average arthritis index significantly was significantly reduced, which was significantly different from that of the CIA model group.

**Table 4. Effect of isoferulic acid and Cimicifugae rhizoma on arthritis index of CIA arthritis mice (MEAN ± SEM)**

| | Day 0 | Day 3 | Day 6 | Day 9 | Day 12 | Day 15 |
|---|---|---|---|---|---|---|
| Normal control group | 0±0 | 0±0 | 0±0 | 0±0 | 0±0 | 0±0 |
| CIA group | 0±0 | 0.57±0.28 | 2.71±0.94** | 4.57±0.90* ** | 7.43±0.75*** | 8.43±0.9 6*** |
| Cimicifuga e rhizoma group | 0±0 | 0.43±0.40 | 1.0±1.41 | 2.29±0.60 | 3.71±0.83^{##} | 4.43±1.0 3^{#} |
| Isoferulic | 0±0 | 0.43±0.28 | 1.0±0.61 | 3.14±0.77 | 5.14±0.98^{#} | 5.71±0.8 |
| acid group | | | | | | 3^{#} |

| | | | | | | |
|---|---|---|---|---|---|---|
| Note: * represents a statistical difference between the CIA group and the normal control group, **, p <0.01; ***, p <0.001. # represents a statistically significant difference between the medication group and the CIA group, #, p <0.05; ##, p <0.01. | | | | | | |

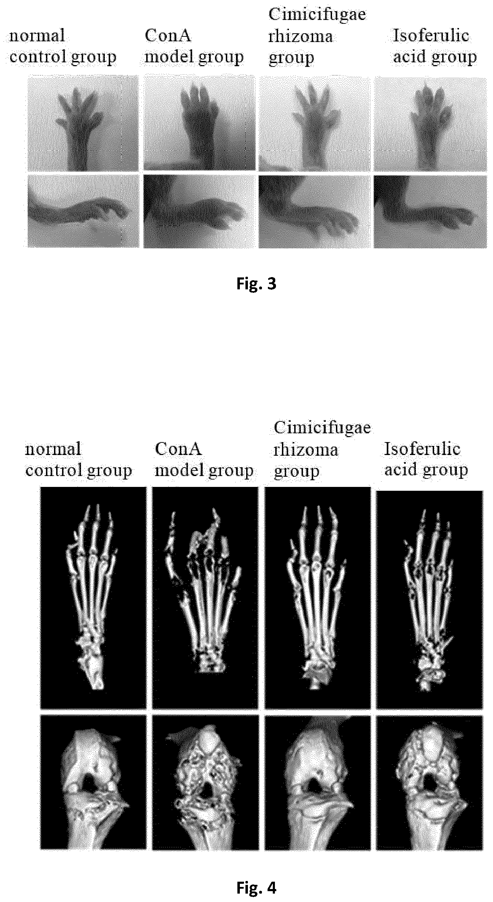

Figure 3 shows pictures of swelling of the front and hind paw joints of mice in the normal group, the CIA model mouse, and the medication group. The picture shows that the swelling of the ankle and toe joints of CIA arthritis mice was obvious, and the swelling of the mice in the isoferulic acid and Cimicifugae rhizoma group was significantly reduced. The results show that both isoferulic acid and Cimicifugae rhizoma can improve joint redness and swelling and dysfunction in CIA arthritis mice.

### Experimental Example 4. Effects of Isoferulic Acid and Cimicifugae rhizoma on Body Weight of CIA Mice

The test materials and animals were the same as those in Experimental Example 3. Model preparation and administration: same as in Experimental Example 3. On day 21 of the second immunization, the mice were weighed.

Results: as shown in Table 5, there was no difference in the average weight of mice among the groups. The results show that isoferulic acid and Cimicifugae rhizoma did not affect the body weight of CIA arthritis mice.

**Table 5. Effects of Isoferulic Acid and Cimicifugae rhizoma on the Weight of CIA Arthritis Mice (MEAN ± SEM)**

| | Body weight (g) |
|---|---|
| Normal control group | 21.9±1.3 |
| CIA group | 21.5±0.9 |
| Cimicifugae rhizoma group | 20.9±0.6 |
| Isoferulic acid group | 21.6±0.4 |

### Experimental Example 5. Effects of isoferulic acid and Cimicifugae rhizoma on organ index of CIA mice

The test materials and animals were the same as those in Experimental Example 3. Model preparation and administration: same as in Experimental Example 3. On day 21 of the second immunization of the mice, they were sacrificed by anesthesia, and brain, heart, liver, spleen, lung, kidney, and testis were taken. Placed in a 6-well plate. Each mouse's organs were placed in a hole, quickly weighed, and the organ index was evaluated.

Results: As shown in Table 6, the spleen of the immune organs was used as a representative calculation of the organs taken. Compared with the normal group, the average organ index of the CIA mouse group increased, while the average organ index of the isoferulic acid group and the Cimicifugae rhizoma group did not change compared with the CIA mouse group. The results showed that isoferulic acid and Cimicifugae rhizoma did not affect the average spleen index of CIA mice.

**Table 6. Effects of Isoferulic Acid and Cimicifugae rhizoma on Organ Index of Mice with CIA Arthritis (MEAN ± SEM)**

| | Spleen index (mg) |
|---|---|
| Normal control group | 83.3±29.8 |
| CIA group | 167.6±57.1 |
| Cimicifugae rhizoma group | 167±24.9 |
| Isoferulic acid group | 168.6±19.9 |

### Experimental Example 6. Treatment of CIA arthritis mice with isoferulic acid and Cimicifugae rhizoma

Test materials and animals: Consistent with Experimental Example 3. Anti-type II collagen antibody ELISA kit was purchased from Chondrex, Cytokine & Chemokine 36-Plex Mouse ProcartaPlex ™ Panel 1A cytokine and chemokine detection kit was purchased from ThermoFisher.

Model preparation and drug administration: the drug dosage of each treatment group was the same as that of Experimental Example 3. After 21 days of the second immunization, the mice were anesthetized.
(1) Micro-CT technology was used to examine the effect of each drug group on the joint structure of CIA mice. After taking 3 mice from each group, the right hind limbs were taken immediately after neck dissection and fixed in 4% neutral formalin. After 2-3 days of fixation, a micro-CT scan was performed using a live animal imaging system. Extraction site: extracted the data of the area above the tibial plaque plate of the right hind limb of the mouse. 3 sets of data were taken. Size selection: the upper part of the tibial plaque plate (0.25 * 0.25 * 0.25); measurement indicators of the extracted bones: bone density, bone volume, bone volume fraction and number of bone trabeculae.
(2) The effects of each drug group on the anti-type II collagen antibody content in the serum of CIA mice were measured. Seven mice from each group were examined; peripheral blood was collected and separated from serum. Anti-type II collagen antibodies were detected with Elisa.
(3) The effects of each drug group on cytokine secretion in serum and joint tissues of CIA mice were measured. Seven mice from each group were examined; peripheral blood was collected and separated from serum. Cytokines in serum were detected by multiple factors: IFN-γ, IL-6, IL-9, IL-12, IL-17A, IP-10, MCP-1, MCP- 3. MIP-1α, MIP-1β, Eotaxin, and G-CSF; the right hind limb knees, joints and claws were taken and stored in liquid nitrogen for quick freezing, then homogenized and extracted protein. Multi-factor detection of cytokines in tissues: IL-9, IL -18 and MIP-2 secretion.

Results (1): Micro-CT technology was used to examine the effects of drugs in each drug group on the joint structure of CIA mice.

Results: As shown in Figure 4, the ankle joint structure of CIA model mice was blurry, osteoporosis was present, and the bone was even severely damaged. Although the joint structure of the medication group had different degrees of damage, the damage was significantly lighter than that of the model group, especially the bone damage was significantly lighter. In the model group, the degree of bone erosion was the smallest in the Cimicifugae rhizoma group, followed by the isoferulic acid group.

As shown in Table 7, compared with the normal group, the bone density, bone volume, bone volume fraction, and number of trabeculae of the mice in the CIA group were all reduced. Compared with the CIA model group, the bone mass of the isoferulic acid and Cimicifugae rhizoma groups was significantly increased. The results show that isoferulic acid and Cimicifugae rhizoma can effectively inhibit and relieve joint destruction.

**Table 7. Effects of Isoferulic Acid and Cimicifugae rhizoma on Bone Damage in CIA Arthritis Mice (MEAN ± SEM)**

| | Bone density (mg /cc) | Bone volume (mm^ 3) | Bone volume fraction | number of trabeculae (1/ mm) |
|---|---|---|---|---|
| Normal | 39.7±9.8 | 0.0064±0.0007 | 0.306±0.060 | 7.113±0.651 |
| control group | | | | |
| CIA group | 4.1±1.9*** | 0.0002±0.0001* ** | 0.047±0.032*** | 0.977±0.319*** |
| Cimicifugae rhizoma group | 24.3±5.9^{##} | 0.0025±0.0005^{#} # | 0.134±0.027^{#} | 3.376±0.566^{##} |
| Isoferulic acid group | 9.5±1.5^{#} | 0.0014±0.0005^{#} # | 0.095±0.034^{#} | 2.042±0.572 |

| | | | | |
|---|---|---|---|---|
| Note: * represents a statistical difference between the CIA group and the normal control group, **, p <0.01; ***, p <0.001. # represents a statistically significant difference between the medication group and the CIA group, #, p <0.05; ##, p <0.01. Results (2): The effect of the drug on the serum anti-type II collagen antibody content in CIA mice. | | | | |

The results are shown in Table 8. Compared with the normal group, the secretion of anti-type II collagen antibodies was significantly increased in the CIA group, with a statistical difference (***, p <0.0001). Both the isoferulic acid group and the Cimicifugae rhizoma group significantly reduced the secretion of anti-type II collagen antibodies in the serum (###, p <0.0001).

**Table 8. Effects of isoferulic acid and Cimicifugae rhizoma on anti-type II collagen antibody content in CIA arthritis mice**

| | Anti-type II collagen antibody (Units/ml) |
|---|---|
| Normal control group | 17±2 |
| CIA group | 15797±337*** |
| Cimicifugae rhizoma group | 10830±460^{###} |
| Isoferulic acid group | 9610±664^{###} |

| | |
|---|---|
| Note: * represents a statistical difference between the CIA group and the normal control group, **, p <0.01; ***, p <0.001. # represents a statistically significant difference between the medication group and the CIA group, #, p <0.05; ##, p <0.01. Results (3): The effects of drugs on cytokine secretion in serum and joint tissues of CIA mice were examined. | |

The results are shown in Table 9. Compared with the CIA group, the serum cytokines IFN-γ, IL-12, IL-17A, IL-6, IL-9, chemokines IP-10, MCP -1, MCP-3, MIP-1α, MIP-1β, Eotaxin and colony-stimulating factor G-CSF secretion levels were significantly reduced, with statistical differences, in the isoferulic acid group; meanwhile, the secretion levels of IL-18, IL-9 and MIP-2 in joint tissue were also significantly reduced with statistical differences. The results show that the isoferulic acid group significantly reduced the secretion of multiple inflammatory cytokines in serum and joint tissues.

**Table 9. Effect of isoferulic acid on serum and joint tissue cytokines in CIA arthritis mice (MEAN ± SEM)**

| | Normal control group | CIA group | Isoferulic acid group |
|---|---|---|---|
| IFN-γ(pg/ml) | 15.4±4.3 | 315.5±80.8 | 120.6±37.1^{# ##} |
| IL-12(pg/ml) | 19.2±2.2 | 46.6±15.7 | 21.9±3.5^{##} |
| IL-17A(pg/ml) | 2.5±1.8 | 18.3±5.5 | 4.9±3.1^{###} |
| IL-6(pg/ml) | 2.4±0.8 | 261.3±90 | 23.3±21.6^{###} |
| IL-9(pg/ml) | 10.1±4.2 | 34.7±16.3 | 15±5.8^{#} |
| IP-10(pg/ml) | 66.2±41.5 | 149.1±66.9 | 52.8±20.8^{#} |
| MCP-1(pg/ml) | 42.2±1.1 | 251.3±29.9 | 139.3±43.4^{#} # |
| MCP-3(pg/ml) | 191±84.8 | 306.9±72.4 | 151.9±20^{##} |
| MIP-1α(pg/ml) | 2.6±0.5 | 8.5±5 | 2.4±0.6^{#} |
| MIP-1β(pg/ml) | 4.1±0.5 | 8.3±2.9 | 3.2±1.1^{##} |
| Eotaxin(pg/ml) | 455.6±54.8 | 568.3±131. 3 | 349.6±85.4^{#} |
| G-CSF(pg/ml) | 2.4±1 | 41.2±11.7 | 4±1.2^{###} |
| Joint tissue IL-18(pg/mg) | 62.3±38.2 | 325.3±141. 8 | 111.1±125^{#} |
| Joint tissue IL-9 (pg/mg) | 3.4±0.6 | 11.2±7.2 | 3.2±1.5^{#} |
| Joint tissue MIP-2 (pg/mg) | 1.3±0.5 | 391.4±253. 3 | 61.2±45.8^{#} |

| | | | |
|---|---|---|---|
| Note: # represents a statistical difference between the medication group and the CIA group, #, p <0.05; ##, p <0.01; ###, p <0.001. | | | |

### Experimental Example 7. Effect of isoferulic acid and Cimicifugae rhizoma on the proportion of granulocytes in CIA arthritis mice

Test materials and animals: CD48 PE and CD11b FITC antibodies were purchased from BD Pharmingen; RBC Lysis Buffer was purchased from Biolegend of the United States; other required reagents were the same as in Experimental Example 3.

Model preparation and drug administration: the administration dose of each treatment group was the same as that of Experimental Example 3. 21 days after the second immunization, the mice were anesthetized.

Animal: C57BL/6 mouse, purchased from Beijing Huafukang Biotechnology Co., Ltd.

Preparation of bone marrow single cell suspension: After anesthesia of C57BL/6 mice, the two pairs of femur and tibia of the mouse were separated, PBS was pipetted with a 2 mL syringe to flush the bone marrow cells into a flow tube, and pipetted repeatedly to obtain the bone marrow single cell suspension. The bone marrow was filtered into a flow tube with a 400-mesh strainer, discarded the supernatant after centrifugation, added 2 mL of RBC Lysis Buffer and lysed at room temperature for 15 min. Discarded the supernatant by centrifugation, harvested the bone marrow cells, resuspended by adding 1 mL of PBS, and repeatedly mixing by pipetting 100 µL into the flow tube for surface staining.

Preparation of single cell suspension of spleen: After anesthesia of C57BL/6 mice, isolated the spleen of the mouse, placed the spleen in a small dish with 4 ml of PBEB added, grinded the spleen with a matte surface of a glass slide, and the spleen cells were filtered into a flow tube with a 400-mesh strainer. The supernatant was discarded after centrifugation. After adding 2 mL of RBC Lysis Buffer to lyse at room temperature for 15 minutes, the supernatant was discarded by centrifugation, and spleen cells were harvested. Then added 1 mL of PBS for resuspension, and repeatedly pipetting and mixing. Pipetted 100 µL into a flow tube for surface staining.

Surface staining: added 0.5 µL of antibody CD48-PE and 1 µL of CD11b-FITC, protected from light, incubated at 4°C for 15 minutes, added 2 mL PBEB and centrifuged, discarded the supernatant. Flow cytometer BD Calibar was used to measure the percentage of CD48-CD11b⁺ positive granulocytes to white blood cells.

Results: As shown in Table 10, in the bone marrow and spleen, the proportion of granulocytes in CIA arthritis mice increased, and the proportion of granulocytes in the bone marrow and spleen were reduced in the isoferulic acid group; the proportion of granulocytes in the spleen was reduced in the Cimicifugae rhizoma group. The reduction was statistically different. The results show that: isoferulic acid and Cimicifugae rhizoma reduced the proportion of granulocytes in CIA arthritis mice.

**Table 10. Effects of Isoferulic acid and Cimicifugae rhizoma on the proportion of granulocytes in CIA arthritis mice (MEAN ± SEM)**

| | Bone marrow (% ) | spleen (% ) |
|---|---|---|
| Normal control group | 34.4±1.4 | 7±1.9 |
| CIA group | 53.1±5.4 | 13.8±2.5 |
| Cimicifugae rhizoma group | 49.1±3.8 | 9.9±3.5^{#} |
| Isoferulic acid group | 44.6±5.1^{#} | 9±4.4^{#} |

| | | |
|---|---|---|
| Note: # represents a statistical difference between the medication group and the CIA group, #, p <0.05; ##, p <0.01. | | |

### Experimental Example 8. Effects of isoferulic acid and Cimicifugae rhizoma on the proliferation of granulocytes and NK cells in CIA arthritis mice

Test materials and animals: CD3-PerCP, NK1.1-APC antibody, Ki67-PE antibody, Intracellular Fixation & Permeabilization Buffer Set were purchased from eBioscience; CD48 PE, CD11b FITC and B220-FITC antibodies were purchased from BD Pharmingen company; other required reagents were the same as in Example 7.

Model preparation and drug administration: the administration dose of each treatment group was the same as that of Experimental Example 3. 21 days after the second immunization, the mice were anesthetized.

Animal: C57BL/6 mice, purchased from Beijing Huafukang Biotechnology Co., Ltd.

The proliferation capacity of the spleen Ki-67 expression response cell was detected by flow cytometry. Two tubes of spleen single cell suspension were prepared and used to detect the proliferation of CD48⁻CD11b⁺ positive granulocytes and CD3-NK1.1⁺B220⁻ NK cells.

The surface staining procedure was the same as in Experimental Example 7. Intracellular staining: after surface staining, intracellular staining was performed: 1) added 100 µL of fixed membrane-breaking solution Fixation Buffer, protected it from light at room temperature, fixed the membrane for 40 min; 2) added 1 mL of Permeabilization Buffer and washed twice, discarded the supernatant 3) added 1 µL of Ki-67-PE antibody at room temperature and protected it from light for 40min; 4) same as step 2); and 5) used flow cytometer BD Calibar to measure the percentages of intracellular Ki-67 expression in CD48⁻CD11b⁺ positive granulocytes, and CD3⁻ NK1.1⁺B220⁻NK cells, respectively.

Results: as shown in Table 11, compared with normal mice, the proliferation ability of granulocytes in bone marrow and spleen of CIA arthritis mice and the proliferation ability of NK cells in spleen were enhanced, and the proliferation ability of the mice in the isoferulic acid group was significantly reduced. There is a significant difference; compared with CIA arthritis mice, the proliferation ability of NK cells in bone marrow and spleen of the Cimicifugae rhizoma group was evidently reduced, which was significantly different. The results show that: isoferulic acid and Cimicifugae rhizoma reduced the proliferation of granulocytes and NK cells in CIA arthritis mice.

**Table 11. Effects of Isoferulic Acid and Cimicifugae rhizoma on Proliferation of Granulocytes and NK Cells in CIA Arthritis Mice (MEAN ± SEM)**

| | Granulocytes (%) | | NK cells (%) |
|---|---|---|---|
| | bone marros | spleen | spleen |
| Normal control group | 29.3±18.9 | 9.9±2.1 | 16.4±2 |
| CIA group | 56±12.1 | 31.2±8.6 | 35±19 |
| Cimicifugae rhizoma group | 29.9±12.4^{#} | 17.6±5.1 | 8.9±1.7^{##} |
| **Isoferulic** acid group | 27.8±8.2^{##} | 26.8±12.5^{##} | 14±9^{#} |

| | | | |
|---|---|---|---|
| Note: # represents a statistically significant difference between the medication group and the CIA group, #, p <0.05; ##, p <0.01. | | | |

In summary, Cimicifugae rhizoma compatibility drugs, Cimicifugae rhizoma extract, Cimicifugae rhizoma total phenolic acid and isoferulic acid can significantly improve the survival rate of mice with hepatitis, improve liver function, reduce liver pathological damage, and inhibit the release of inflammation factors, and provide significant liver protection.

Isoferulic acid and Cimicifugae rhizoma can significantly reduce the incidence of CIA arthritis mice, reduce the average index of CIA arthritis, reduce joint swelling, reduce bone erosion and bone destruction, reduce the content of anti-type II collagen antibodies, widely inhibit the release of inflammatory factors, reduce the proportion and proliferation capacity of granulocytes and NK cells, and have a wide range of immunosuppressive effects.

In addition, isoferulic acid, Cimicifugae rhizoma extract, Cimicifugae rhizoma also have evident inflammatory cytokine inhibitory effects.

Unless specifically defined, the terms used in the present invention have the meanings generally understood by those skilled in the art.

The embodiments described in the present invention are for exemplary purposes only and are not intended to limit the protection scope of the present invention. Those skilled in the art can make various other substitutions, changes, and improvements within the scope of the present invention. Therefore, the present invention is not limited to the above-mentioned embodiments and is only limited by the claims.

## Claims

1. Pharmaceutical composition comprising isoferulic acid for use in the treatment of autoimmune hepatitis.

## Patentansprüche

1. Pharmazeutische Zusammensetzung umfassend Isoferulasäure zur Verwendung bei der Behandlung von Autoimmunhepatitis.

## Revendications

1. Composition pharmaceutique comprenant de l'acide isoférulique pour son utilisation dans le traitement d'une hépatite auto-immune.
